# EUROPEAN PATENT APPLICATION

(11) **EP 3 731 240 A1**
(43) Date of publication of application: **28.10.2020**
(21) Application number: 19170866.8
(22) Date of filing: 24.04.2019
(51) Int. Cl.: G16H 80/00, G01R 19/25

(54) **NON-INVASIVE MONITORING FOR ASSISTIVE LIVING SYSTEMS**

(71) Applicant: Intuity Media Lab GmbH, 70176 Stuttgart (DE)
(72) Inventor: KAGERMAN, Franz, 70563 Stuttgart (DE)
(74) Representative: Hoffmann Eitle

(57) **Abstract**

A computer-implemented method for non-invasive monitoring, the method comprising: receiving (S10) first output signals (10) from an electrical meter (100) coupled to an electrical supply of a residence, the first output signals (10) indicating one or more usage parameters related to electrical appliances (110₁, 110₂, ... 110_{N}) connected to the electrical supply; receiving (S20) second output signals (20) from a water meter (200) coupled to a water supply of the residence, the second output signals (20) indicating one or more usage parameters related to water appliances (120₁, 120₂, ... 120_{M}) connected to the water supply; processing (S40) the first (10) and second output signals (20) based on one or more machine learning algorithms to classify individual electrical appliance usage and individual water appliance usage; the first signals having a sampling frequency of at least 10 kHz; and based on the one or more machine learning algorithms, classifying (S50) an activity of a user and/or classifying an event in the residence based on the classified individual electrical and water appliance usage.

## Description

### Technical Field

The present invention relates to a non-invasive monitoring for assistive living systems, and specifically relates to a non-invasive monitoring unit and a computer-implemented method for non-invasive monitoring.

### Background

As the population in industrialized countries ages, the need for decentralized digital health care systems increases. In Europe, the part of the population being older than 65 years increases rapidly and will likely reach 81 million in 2025. A stationary care for all or the patient's wish for a longer stay in their homes cannot be guaranteed with the current systems. There is thus an urgent need for a technological transformation and decentralization of the health system.

An Assistive Living System (ALS) in conjunction with machine learning algorithms enable a classification of daily activities of patients or users in a monitored environment, for example for the purpose of monitoring a daily routine of the patient or user. Here, an activity may be considered in connection with a condition under which behavioral and/or actual activities are performed.

In case of irregularities or emergency situations within an ALS monitored environment, a nurse, a caregiver, a medical professional or the like may examine the irregularities or emergency situations from outside and may initiate and/or take necessary steps.

A conventional ALS may detect specific operation states of electrical appliances of the monitored environment, for example whether an electric appliance is switched on or off, such as that a kitchen stove is switched on, may classify an activity of the patient/user (e.g. "the patient is cooking"), and may thus provide an insight into the daily routine of the patient/user.

### Summary of the Invention

### Technical Problem

A conventional ALS requires, however, a large number of sensors and also cameras in a monitored environment of a user's residence (an apartment, a house, or the like) in order to accurately classify individual daily activities and to generate a context therefrom. Herein, context is to be understood as a relation between daily activities such that an information about the activity of the monitored person and/or an event occurring in the monitored environment can be made. This implies significant costs for purchasing, installation and maintenance of the large number of sensors and cameras.

Moreover, conventional ALSs are highly invasive as the perceived privacy of the user/patient is typically impaired due to the fact that the sensors and cameras are visible objects for the user/patient and also relatives.

There is thus a need to address the above technical problems, in particular to provide an ALS that is less expensive and that avoids an invasive character.

A first step in that direction has been suggested in US 2014/0172758 A1, describing a personal emergency response system by non-invasive load monitoring. In particular, in this system, signals of a non-invasive load monitoring system coupled to an electrical supply of a person's residence are received, the output signals indicating switching events of appliances connected to the electrical supply. A computer processor is then used to process the output signals in accordance with a machine learning algorithm to identify appliance activation routines. Rules are defined based on the identified appliance activation routines, and the computer processor is used to monitor the output signals and apply the rules to the output signals to identify appliance switching conditions that violate the rules.

Further, in WO 2015/124972 A1 a system and a method for non-intrusive monitoring in relation to utility meters in a facility is disclosed. The system primarily uses the data generated by the utility meters, namely electric and water meters, to produce patterns of activities that are performed by an individual. Any sort of discrepancy observed between a newly generated data pattern and previous patterns signals an alarm in the form of a message which is then sent to the concerned person.

Further, in DE 10 2017 000 838 A1 a method for monitoring the activity of at least one person in an infrastructure unit is disclosed, wherein utility meters measuring water and electric usage are used. Consumption profiles over time for a predetermined time period are provided as profile data, which are evaluated by means of at least one activity criterion carrying out a comparison with reference data describing a comparative profile describing a normal activity for the time period.

Such non-invasive ALSs require significantly less costs, at least reduced by a factor of 10 for a conventional ALS being estimated on the assumption of 12 RFID sensors and an on-site control unit (embedded hardware) and including maintenance and installation costs. This cost factor is additive and would scale with each further apartment or house room.

Such a non-invasive ALS also do not scale with the monitored space (number of rooms), is real-time capable, and is non-invasive because the electrical meter and the water meter are not visible for the user/patient inside the residence/apartment. They are typically installed hidden in the basement or the like. This means that the user/patient may follow the normal daily routine without having to activate or operate anything.

However, these conventional approaches for non-invasive monitoring and the corresponding apparatuses have the technical problem that a precise and accurate monitoring and classification of activities is very difficult. Thus, monitoring of smaller, but relevant changes deviating from established routines cannot be monitored with present systems. Such inaccuracies may also cause inappropriate alarms which erroneously indicate that the user/patient requires medical help. While it would be possible to add additional sensors in the user's residence to acquire additional data for improved monitoring, this would generally obstruct the non-invasive character and necessarily increase the hardware costs of the ALS.

Therefore, an alternative mechanism for non-invasive monitoring that enables a more precise and accurate monitoring and activity classification is needed.

### Solution

In this regard, the present inventors have devised, in accordance with a first example aspect herein, a computer-implemented method for non-invasive monitoring, the method comprising the steps of receiving first output signals from an electrical meter coupled to an electrical supply of a residence, the first output signals indicating one or more usage parameters related to electrical appliances connected to the electrical supply; receiving second output signals from a water meter coupled to a water supply of the residence, the second output signals indicating one or more usage parameters related to water appliances connected to the water supply; processing the first and second output signals based on one or more machine learning algorithms to classify individual electrical appliance usage and individual water appliance usage; the first signals having a sampling frequency of at least 10 kHz; and based on the one or more machine learning algorithms, classifying an activity of a user and/or classifying an event in the residence based on the classified individual electrical and water appliance usage.

The present inventors have further devised, in accordance with a second example aspect herein, a computer program which, when executed by a computer, causes the computer to perform the method according to the first example aspects herein.

The present inventors of further devised, in accordance with third example aspect herein, a non-invasive monitoring unit, comprising: a communication module, configured to receive first output signals of a electrical meter coupled to an electrical supply of a residence, the first output signals indicating one or more usage parameters related to electrical appliances connected to the electrical supply; and receive second output signals of a water meter coupled to a water supply of the residence, the second output signals indicating one or more usage parameters related to water appliances connected to the water supply; and a processing module, configured to: process the first and second output signals based on one or more machine learning algorithms to classify individual electrical appliance usage and individual water appliance usage, the first signals having a sampling frequency of at least 10kHz; and based on the one or more machine learning algorithms, classify an activity of the user and/or classify an event in the residence based on the classified electrical and water appliance usage.

The non-invasive monitoring method, the computer program and the corresponding non-invasive monitoring unit of the present invention enable such precise and accurate monitoring by employing at least a smart electrical meter, that has a maximum sampling frequency of at least 10 kHz, and a smart water meter, wherein the smart electrical meter is coupled to an electrical supply of a residence and acquires first output signals indicating one or more usage parameters related to electrical appliances connected to the electrical supply and the smart water meter is coupled to a water supply of a residence and acquires second output signals indicating one or more usage parameters related to water appliance usage.

### Brief description of the drawings

Embodiments of the invention will now be described, by way of nonlimiting examples only, with reference to the accompanying figures described below. Like reference numerals appearing in different ones of the figures can denote identical or functionally similar elements, unless indicated otherwise.
Fig. 1A is a flow chart of a method for non-invasive monitoring in accordance with an embodiment and Fig. 1B illustrates further aspects of the implementation of this method in a residence.
Fig. 2 is a flow chart of a method for non-invasive monitoring in accordance with another embodiment.
Fig. 3 is a flow chart illustrating the step of adapting the used frequency of smart electrical meter in accordance with another embodiment.
Fig. 4 is a schematic diagram of a non-invasive monitoring unit in accordance with another embodiment.
Fig. 5 is a schematic diagram of a non-invasive monitoring unit in accordance with another embodiment.
Figs. 6A - 6D are schematic diagrams of non-invasive monitoring systems comprising of a non-invasive monitoring unit, a smart electrical meter and a smart water meter in accordance with another embodiment.

### Detailed description

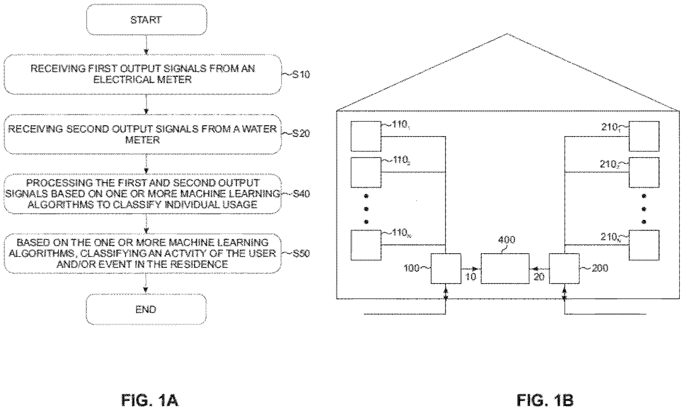

Referring to Fig. 1A, a flow chart of a method for non-invasive monitoring in an Assistive Living system (ALS) is illustrated. This method is implemented by a computer, which may include several realizations such as, for example, a microprocessor, (for example, if the method is implemented in a smart electrical meter), a personal computer, a laptop, a remote cluster, a tablet computer and the like, and is generally referred to as a non-invasive monitoring unit 400 below. Further referring to Fig. 1B, a residence is illustrated in which the non-invasive monitoring for the ALS is implemented.

In step S10 according to Figs. 1A and 1B, first output signals 10 are received or acquired from an electrical meter 100 coupled to an electrical supply of a residence, wherein the first output signals 10 indicate one or more usage parameters related to electrical appliances 110₁, 110₂, ..., 110_{N} connected to the electrical supply. Here, electrical appliances refer to any electricity-consuming appliance such as an electrical oven, a TV, an air conditioning, a computer, electrical lighting, a water boiler and the like.

Note that "output signals" may refer to data or feature data or the like. Further, a "user" may refer to a patient or a subject performing activities within a monitored environment and "residence" may refer to "monitored site" or "monitored environment" or the like. Additionally, note that the ALS may not be limited to a residence for one user only, but may be adapted for more than one user. Distinguishing activities between various users in the same residence could be achieved by an additional step of analyzing the usage parameter with regard to, for example, different time patterns. An example could be recognizing that a certain set of usage parameter always appears in combination at a first time period and has certain distinct differences compared to a similar, but not identical set of activities at a second time period, for exampling using the bathroom from 6 AM to 6:30 AM in the morning and then again using the bathroom from 7 AM to 7:20 AM. From this it could be distinguished that there are two users in the residence. Alternatively, external information might be provided which indicates that there is more than one user in the residence, possible this external information also includes information helping to distinguish different users, such as different habits, different schedules and like.

Further, usage parameters related to electrical appliances may refer to one or more of a switching event (on, off, switch-over), a voltage, a current, a real and a reactive power (for example in a frequency range of 1-50 Hz), harmonics (for example in an frequency range of 50Hz to 10 kHz), an electromagnetic interference (EMI) (for example in an frequency range of 10 kHz), a time, a time stamp, a usage time, a state (on, off, stand-by, maintenance) and the like. Such usage parameter may be determined by the electrical meter 100. Here, the electrical meter 100 maybe a (smart) electrical meter that is connected to the electrical appliances 110₁, 110₂, ..., 110_{N} in the residence and to the electric power company via a power line, records the electric energy consumption based on a variable sampling frequency, and communicates corresponding information to the non-invasive monitoring unit 400. Furthermore, while voltage and electric current may be (directly) measured by the (smart) electrical meter 100 using a selected sampling frequency, other usage parameter such as real and reactive power or harmonics may be derived by the smart electrical meter 100 or the non-invasive monitoring unit 400.

In step S20 according to Figs. 1A and 1B, second output signals 20 are received or acquired from a water meter 200 coupled to a water supply of a residence, wherein the second output signals 20 indicate one or more usage parameters related to water appliances 210₁, 210₂, ..., 210_{M} connected to the water supply. Here, water appliances refer to any water-consuming appliance such as a kitchen sink, a shower, a lavatory or the like.

Usage parameters related to water appliances may refer to one or more of water consumption, a water flow rate, a flow velocity, a time, a time stamp, a usage time, a state (on, off, stand-by, maintenance) and the like. Such usage parameter may be determined by the water meter 200, for example based on ultrasound technology or remanent magnetic field technology. Here, the water meter 200 maybe a (smart) water meter that is connected to the water appliances 210₁, 210₂, ..., 210_{M} in the residence and to the waterworks, records the water consumption based on a variable sampling frequency (as small as minutes), and communicates corresponding information to the non-invasive monitoring unit 400. Furthermore, the water meter 200 may provide precise measurements of flow rates, such as 10 l/h, 1 l/h, 0.1 l/h or even smaller.

Note that this order of steps S10 and S20 is not limiting, in other words, it is conceivable that the two steps are performed in a different order, in a variable order, without any fixed order, or in parallel.

Next, in step S40 according to Fig. 1A, the first and second output signals are processed based on one or more machine learning algorithms to classify individual electrical appliance usage and individual water appliance usage. Here, the first output signals 10 have a sampling frequency of at least 10 kHz. That is, based on the first output signals from the electrical meter 10 and the second output signals from the water meter 20, the usage of individual electrical appliances and the usage of individual water appliances is determined; for example whether a TV is used, whether an electric oven is used, whether a shower is used, whether the lavatory is used and the like. Further, based on the first output signals from the electrical meter 10 having a sampling frequency of at least 10 kHz, operation modes of the individual electrical appliances may be determined; as will be further discussed below.

The sampling frequency may further vary or may be adapted or selected. In order to appropriately take the actual sampling frequency into account when classifying the individual electrical appliance usage, the non-invasive monitoring unit may be provided with current values of the sampling frequency, for example as a further part of the output signals 10, or may derive the actual sampling frequencies from data entries of the output signals 10, such as the time stamps.

Here, the electrical meter 100 may be a high-frequency non-instructive load monitoring smart meter (HNSM) for tracking electrical appliance usage within the monitored site (residence). Such an electrical meter 100 is capable of using, at least temporarily, a sampling frequency of at least 10 kHz (as will be further described below) . In particular, the smart electrical meter 100 may select a sampling frequency, for example 10 kHz, 50 kHz, 100 kHz depending on an input instruction provided by the non-invasive monitoring unit 400. As such, the sampling frequency may dynamically be selected or adapted, for example according to different conditions, as will be further described below. Here, the usage of such sampling frequencies allows for a suitable and improved classification of an individual appliance usage and therefore an improved classification of the user activity. More specifically, these higher sampling frequencies not only allow to distinguish/classify between individual electrical appliances, but also allow to distinguish/classify, for example, between different operating modes of an electrical appliance, e.g. different operating modes of an electrical oven (cooking, baking, grilling), different operating modes of a TV (frequent channel switching, internet usage, radio channel usage, TV station channel usage), and the like.

Note that the one or more machine learning algorithms themselves may be provided externally from the non-invasive monitoring unit 400. In particular, training, acquisition of training data (e.g. usage parameter as described above), and the like may be provided in an external data server (not shown) which provides a trained classification algorithm to the non-invasive monitoring unit 400. In other words, the non-invasive monitoring unit 400 may apply a trained classification algorithm in step S40 to classify the individual electrical appliance usage and/or individual water appliance usage.

Classification of individual electrical appliance usage based on one or more machine learning algorithms means that the acquired data of individual electrical appliance usage may be classified in such a way that non-invasive information is derived as to a particular usage of one or more electrical appliances by the user. In the simplest case, this might be realized by classifying the individual electrical appliance usage according to predefined categories such as "no electrical appliance usage", "low electrical appliance usage", "high electrical appliance usage", or categories such as "periodic/repeating electrical appliance usage", but it is not limited thereto. In a more advanced case this might lead to classification "electrical appliance usage typical for a TV, further indication towards use via laptop", which is only possible through the high sampling frequency provided by the high frequency smart electrical meter. If the used sampling frequency is high enough it is not only possible to deduce which devices are used but also in which operation mode they are used, such as which TV station is tuned into, etc. providing further information that is potentially crucial in order to determine activity (or non-activity) of a user. Consequently, categories such as "usage of device X", "usage of device X in combination with device Y", "usage of device X in mode Z" and the like could be determined. Further, the classification is not limited to the case of predefined categories. In particular, new categories might be added to the list of categories, for example if the usage parameters related to electrical appliances indicate parameters related to one or more of voltage, current, real and reactive power, harmonics, electromagnetic interference that appear to be related to a new electrical apparatus. Here, such usage parameter may be known to the machine learning algorithm / trained classification algorithm (for example, if trained in an external server), and thus detect a specific electrical apparatus that is newly purchased by the user and is used for the first time in the residence. Further, categories may be deleted if deemed necessary, for example if the categories are not used (any more) and/or different categories exist that provide better results. Further, if unknown usage parameters of an electrical appliance are detected, this may indicate a potential hazardous condition and the user or remote caregiver may be warned accordingly.

Similarly, classification of individual water appliance usage based on one or more machine learning algorithms means that the acquired data of individual water appliance usage may be classified in such a way that non-invasive information is derived as to a particular usage of one or more water appliances by the user. This might be realized by classifying the individual electrical appliance usage according to predefined categories such as "no water appliance usage", "low water appliance usage", "high water appliance usage", or categories such as "periodic/repeating water appliance usage", but it is not limited thereto. Further, also this classification is not limited to the case of predefined categories. In particular, new categories might be added to the list of categories, for example. In a more advanced case this might lead to classification "water appliance usage typical for a bathtub" or the like, possible through the higher sampling frequency provided by the smart water meter. In addition, classifications such as "water appliance usage typical for a bathtub and a sink" might be possible as well. Consequently, categories such as "usage of device X", "usage of device X in combination with device Y" and the like could be determined. Also here categories may be deleted if deemed necessary, for example if the categories are not used and/or different categories exist that provide better results. Further, if usage parameters of a water appliance indicate water leaks, reverse water flows, or the like, this may also indicate a potential hazardous condition and the user or remote caregiver may be warned accordingly.

Furthermore, the classification may be combined in such a way that the usage parameters of both, electrical and water, appliance are combined. Examples for this might be a classification such as "water appliance usage typical for a bathtub" and "electrical appliance usage typical for an electrical stove". Such a classification may provide an improved classification of an activity or event (as will be further described below) . Such a classification may also be useful as an indication that something is not in order, for example that the bathtub might overflow the bathroom while the user is cooking in the kitchen or that on the stove food is burning while the user is taking a bath. These classifications are only possible with a high frequency electrical meter as only with such a high frequency the classification can be made accordingly. Without a high frequency electrical meter, the use of electricity and water at the same time, such as in the above example, could not be distinguished from a normal and unproblematic case such as "water appliance usage typical for a bathtub and electrical appliance usage typical for a radio", which would rather point to normal, harmless use of a listening to a radio while taking a bath.

In the last step S50 depicted of the method as illustrated in Fig. 1A, based on the one or more machine learning algorithms, an activity of the user and/or an event in the residence is classified, based on the classified electrical and water appliance usage.

Classification of an activity based on one or more machine learning algorithms means that the machine learning algorithm or a classification algorithm is able to deduce from the classification of the electrical appliance usage and from the classification of the water appliance usage that a certain activity using devices using electricity and/or water is performed. Activity of a user may generally refer to the usage and the change in the usage of a device. This can possibly include the usage of a combination of devices. As an example, such a classification might be, regarding electricity using devices, "Person is using the TV" or "Person is not using the computer anymore" or "Person switched on the lights in the bathroom", or, regarding water using devices, "Person fills the bathtub", or "Person empties out the water in the sink" or "Person uses the toilet", whereas activities might also include examples combining devices, such a "using the shower and using the radio" or "using the bathtub and the electrical stove".

Similarly, classification of an event based on one or more machine learning algorithms means that the machine learning algorithm is able to deduce from the classification of the electrical appliance usage and from the classification of the water appliance usage that a certain event involving devices using electricity and/or water is occurring. As an example, such a classification might be, regarding electricity using devices, "the light in the bedroom is still on", "the stove is still on and it is past midnight", or, regarding water using devices, "the water in the bathtub is still flowing so it might overflow" and the like.

As outlined above, the use of smart meters, in particular of a smart electrical meter with a high sampling frequency can be used to support old, handicapped or sick people, in particular regarding the use of places that are prone to accidents, such as the bathroom and the kitchen, which are particularly important. Also research can profit from such a system as it could lead to a better understanding of the causes leading to dementia and further possibly even provide support in predicting the onset of dementia.

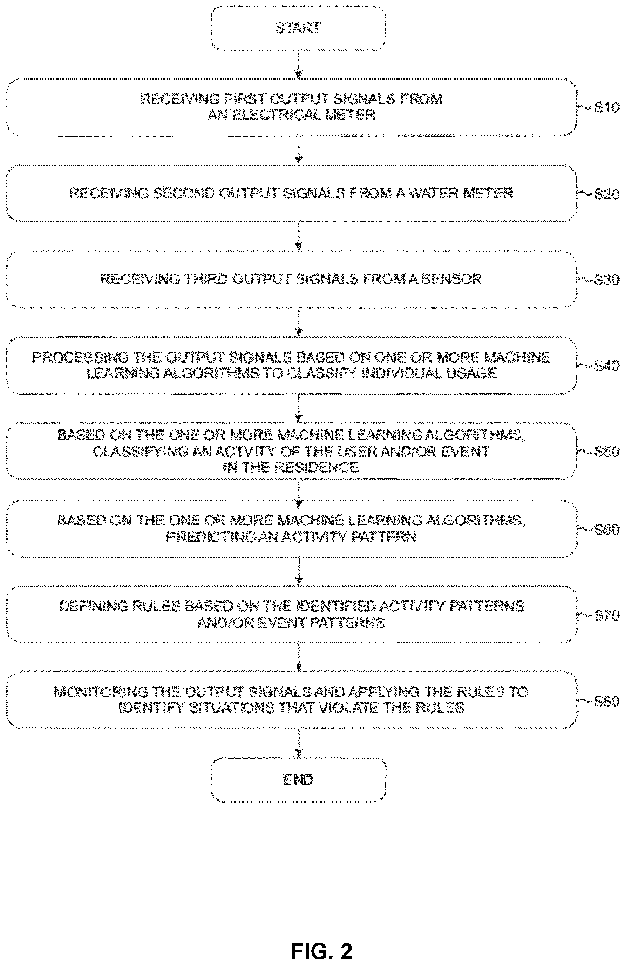

Referring to Fig. 2, a flow chart of a method for non-invasive monitoring according to another embodiment is illustrated. As explained above, this method is implemented by a computer, which may include several realizations such as, for example, a microprocessor, a personal computer, a laptop, a remote cluster, a tablet computer and the like, and is generally referred to as a non-invasive monitoring unit below.

As Fig. 2 includes steps that are also present in Fig. 1A, namely steps S10, S20, S40, and S50, these steps are not explained in further detailed; rather it is referred to the description of Fig. 1A.

In addition to Fig. 1A, Fig. 2 includes an optional step S30, positioned before step S40 and after the steps S10 and S20. In this step S30 according to Fig. 2, third output signals 30 may be received or acquired from a sensor 300 of a residence. These third output signals 30 may, for example, refer to a gas appliance usage. These third output signals 30 may also refer to position information of the user, for example based on wearable devices which the user wears around the arm, the wrist, the neck or the like. Alternatively, the third output signals 30 may refer to radar sensors.

Here, based on the one or more machine learning algorithms, a context of the user's activity is generated. Generating a context of the user's activity means that a connection or relationship is determined between activities and/or events classified in step S40. This might be done based on a combination of categories (as explained above), such as common combinations of activities of a user that can be categorized, such as "preparing and eating breakfast while listening to the radio" or "watching TV after dinner" as well as "taking a bath and preparing to sleep", but is not limited thereto. In particular, the machine learning algorithm might be able to generate context among several classified activities beyond the predefined categories and/or might not rely on predefined categories at all. An illustrative example for the generation of context is as follows: "User is using the stove (Time, Duration; as detected by the electrical meter), water boiler (Time, Duration; as detected by the water meter) and watches a certain TV Series (Time, Duration; as detected by the electrical meter). He is making bacon, eggs and a coffee and is therefore having breakfast for the next 30min (Context 1)"; further based on past data, it is possible to determine, with a specific certainty, that the user is going to watch television after breakfast for the next 3h (Context 2). Based on this pattern it is further possible to deduce that the user leads a healthy lifestyle (Context 3).

In step S60 according to Fig. 2, based on the one or more machine learning algorithms, an activity pattern of the user and/or an event pattern in the residence is predicted.

Predicting an activity or event pattern means that from a plurality of classified activities or events, based on the one or more machine learning algorithms, a typical series of activities or events at a specific time, such as daily or weekly, and at a specific place, such as in a specific room in the residence, of a routine of the user is determined. This may include a typical order in a series of activities or events that represent a typical behavior of the user and/or a typical series of events occurring in the residence during the course of the day. This might include activities or events that occur at a specific time and place. An example of this might be "User gets up at 7 am, goes to the bathroom for 15 minutes and then has breakfast for half an hour." or alternatively "User gets up at 7 am, goes to the bathroom for 15 minutes and then has breakfast for half an hour. He is expected to watch television for one hour afterwards."

For this pattern recognition, predefined patterns may be used which may include predefined patterns that have a certain degree of freedom suitable to be fit to the classified activities/events. An example for this might be "User gets up at XX am, goes to the bathroom for YY minutes and then has breakfast for ZZ minutes." Here, the specific values for XX, YY, and ZZ are monitored over an extended period of time, for example several months. This allows for more variability and therefore predicted patterns that fit better to the user's behavior.

Further, the patterns may be updated regularly, that is weekly, daily, hourly or even more frequent, in order to provide optimal monitoring capabilities. Updating patterns might be due to newly acquired classification results, contexts or to alternative, external reasons such as a command by the user.

In step S70 according to Fig. 2, based on the identified activity patterns and/or event patterns, rules are defined.

This step might include specific rules such as "the user gets up by 9am" or might include more flexible rules including time intervals, usage intervals, or the like, such as "the user gets up by between 7 and 8 am", "the user showers for 20 to 30 minutes". The rules may therefore include time conditions, that is at what time an activity or event typically occurs, and/or usage conditions, that is which and how one or more appliances are used. Then, based on the machine learning algorithm, it is possible to dynamically select among several rules that fit best to describe a normal or typical course of activities/events. That is, distinguishing between activities/events at different times may lead to different rules to be applied. For example, getting up at 7 am may typically lead to a different daily routine (i.e. a different rule) then getting up at 9 am.

Further, in step S80 according to Fig. 2, the output signals 10 and 20 are monitored and the rules are applied to these monitored output signals in order to identify situations that violate the rules. Such situations may include hazardous situations, emergency situations and the like. That is, having identified the rules based on a series of previous output signals from the electrical meter 100, the water meter 200, and optionally a sensor 300, the non-invasive monitoring unit subsequently compares current output signals with time conditions, usage conditions and the like defined by the rules in order to determine whether the user(s) follow a typical routine or not. Not following a typical routine may indicate an abnormal or unusual behavior which may indicate an emergency situation which requires a check by a caregiver or a medical doctor.

In addition, the optional third output signals may be used in one or more of the above steps S40 - S80, that is to classifying the activity and/or event, to predict the activity and/or event pattern, and to define the rules. This additional usage of sensor data further improves the classification and non-invasive monitoring capabilities of the ALS.

The method may optionally transmit data generated by any of the steps S40, S50, S60, and S70 to a remote server, for example a cloud server. The remote server (not shown) may implement the one or more machine learning algorithms, and receiving the data from a plurality of residence may improve the training of an actual classification algorithm that may subsequently be implemented or updated at local non-invasive monitoring units. Here, the transmitted data may be prepared by a processing module 420 (to be explained below) of the non-invasive monitoring unit 400 in a way that is usable by the machine learning algorithm(s). In particular, the data may be transmitted via a dataset avoiding empty and inconsistent data entries which improves the machine learning process. The data transmitted might thus allow for further improvement of the machine learning algorithm(s). Data transmission may occur over a cloud infrastructure, over a private or public network and the like, and may be based on, for example, private and public keys, in order to provide data security. In addition, the user of the residence may select, for example via a graphical user interface, which of the data are to be transferred to the remote server in order to provide a data privacy mechanism.

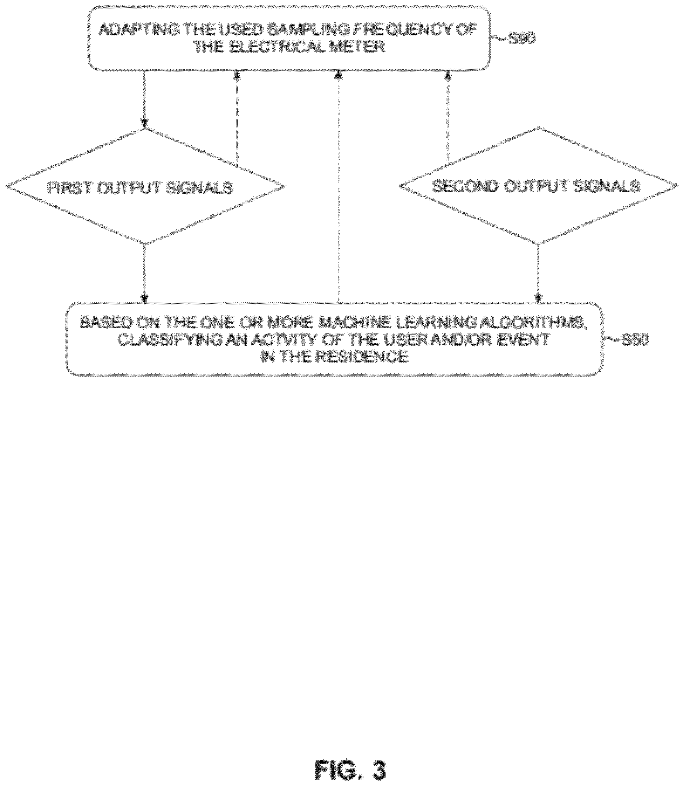

In Fig. 3 a further aspect of the present invention is depicted. Fig. 3 shows an embodiment of how the used sampling frequency of the electrical meter 100 is adapted. Adapting the used sampling frequency of the electrical meter 100 changes the sampling frequency of the first output signals that are used in the further steps as described herein. Adapting the used sampling frequency is useful because adapting high sampling frequencies of at least 10 kHz generate high numbers of data records which leads to disadvantageous data storage requirements.

This step of adapting the used sampling frequency of the electrical meter 100 can be influenced by various sources. Fig. 3 schematically illustrates examples of such sources, namely the adapting or selection of the sampling frequency according to or based on the first and/or second output signals. In particular, the first and/or second output signals themselves may indicate that the usage of electricity and/or water is very low (indicating, for example, that the user is presently not in the residence) so that a classification or monitoring is not necessary. In such a case, the sampling frequency may be dynamically reduced, in particular to values below 10kHz, in order to minimize the number of data records. On the other hand, if the first and/or second output signals indicate that the usage of electricity and/or water starts to increase (again), for example when a user has returned to the residence and switches on a light and/or washes hands, then the sampling frequency may be dynamically increased in order to continue the classification and monitoring steps.

Alternatively, or additionally, the adapting or selection of the sampling frequency may also be based on a classified activity or a classified event. For example, a specific activity (for example in an activity pattern) may indicate that the user will continue performing this activity for an extended period of time, such as typically watching TV between 7 PM and 9 PM. According to such a specific activity, no further activity is typically expected for the user, so that the sampling frequency may be dynamically reduced in order to minimize the number of data records.

Note however, that it is not limited to these, as other sources may be considered also. Examples of such other sources not depicted in Fig. 3 are the specific time of a day (day/night, morning/midday/evening, etc.), established or predetermined routines of the user, alternative information acquired through other means, such as the presence of an additional person, that can have a considerable impact on the electrical and/or water supply and the capabilities of the method in general. During the night, for example, from 11 PM - 6 AM the following morning, the user typically sleeps so that it is not useful to keep the sampling frequency high. The sampling frequency may then be increased (again) around the typical wake-up time of the user, or based on a series of classified activities around a morning routine of the user.

The above adaptation or selection of the sampling frequency according to Fig. 3 thus provides a feedback mechanism with regard to a dynamic variation of the sampling frequency that reduces the storage requirements and applies sampling frequencies of at least 10 kHz only in times of relevant user activity or to times when monitoring of the ALS is required.

Thus, the sampling frequency and thus the amount of generated of data, that have to be processed and potentially stored, can be adapted to the identified needs of the monitoring unit such that the amount of data generated can be reduced, thereby improving performance of the unit, and possibly reducing the size of the unit due to lower data storage requirements and therefore maintaining the feeling of non-invasiveness monitoring that is of central importance for such a system.

This step can be realized either directly in the electrical meter (for example, if the non-invasive monitoring unit is implemented in the electrical meter) or, if necessary, by a module of the non-invasive monitoring unit outside of the electrical meter.

Possible mechanisms how to adapt the sampling frequency (by a corresponding up-sampling and/or down-sampling have already been established. For example, on the basis of the technique described in EP 3 082 078 A1, an appropriate granularity level of the usage data (e.g. in the form of a time-series data) may be selected and applied to produce down-sampled usage data. This can in particular be realized without compromising the privacy of the user and further allowing the costumer to flexibly control the amount of information shared externally. Specific examples might include down-sampling by implementation of a binary-tree based scheme, e.g. a modified Merkle hash tree, or by implementation of a N-ary-tree (instead of binary) based scheme in order to achieve granularity of the data by means of subsequent hash value manipulation. This step can be made for various uses, e.g. data reduction for improved data transfer and improved data storage, feedback and privacy concerns. The up-sampling and/or down-sampling may also be achieved by other means known to the skilled person.

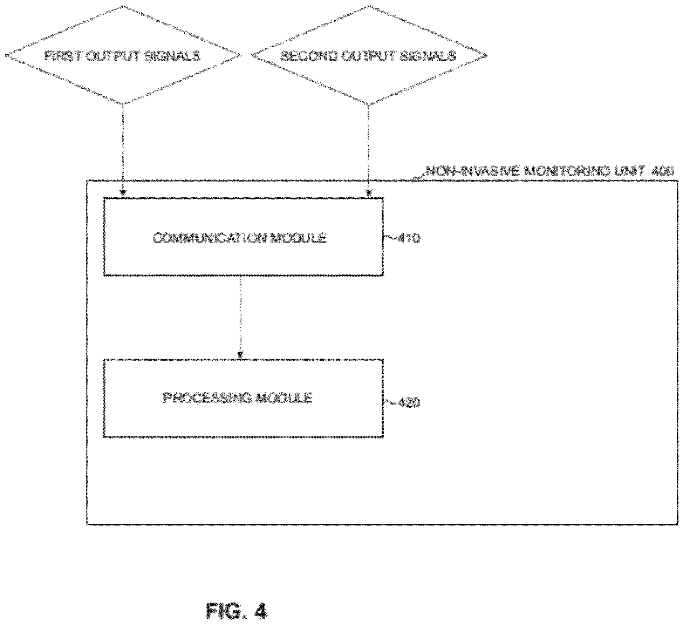

In Fig. 4 an example of a non-invasive monitoring unit 400 according to the present invention is illustrated.

This non-invasive monitoring unit 400 as depicted in Fig. 4 comprises a communication module 410 and a procession module 420.

The communication module according to Fig. 4 is configured to receive or acquire (S10) first output signals 10 from an electrical meter 100 coupled to an electrical supply of a residence, wherein the first output signals 10 indicate one or more usage parameters related to electrical appliances 110₁, 110₂, ..., 110_{N} connected to the electrical supply. Here, electrical appliances refer to any electricity-consuming appliance such as an electrical oven, a TV, an air conditioning, a computer, electrical lighting, a water boiler and the like.

Note that "output signals" may refer to data or feature data or the like. Further, a "user" may refer to a patient or a subject performing activities within a monitored environment and "residence" may refer to "monitored site" or "monitored environment" or the like. Additionally, note that the ALS may not be limited to a residence for one user only, but may be adapted for more than one user. Distinguishing activities between various users in the same residence could be achieved by an additional step of analyzing the usage parameter with regard to, for example, different time patterns. An example could be recognizing that a certain set of usage parameter always appears in combination at a first time period and has certain distinct differences compared to a similar, but not identical set of activities at a second time period, for exampling using the bathroom from 6 AM to 6:30 AM in the morning and then again using the bathroom from 7 AM to 7:20 AM. From this it could be distinguished that there are two users in the residence. Alternatively, external information might be provided which indicates that there is more than one user in the residence, possible this external information also includes information helping to distinguish different users, such as different habits, different schedules and like.

Further, usage parameters related to electrical appliances may refer to one or more of a switching event (on, off, switch-over), a voltage, a current, a real and a reactive power, harmonics, an electromagnetic interference, a time, a time stamp, a usage time, a state (on, off, stand-by, maintenance) and the like. Such usage parameter may be determined by the electrical meter 100. Here, the electrical meter 100 maybe a (smart) electrical meter that is connected to the electrical appliances 110₁, 110₂, ..., 110_{N} in the residence and to the electric power company via a power line, records the electric energy consumption based on a variable sampling frequency, and communicates corresponding information to the non-invasive monitoring unit 400. Furthermore, while voltage and electric current may be (directly) measured by the (smart) electrical meter 100 using a selected sampling frequency, other usage parameter such as real and reactive power or harmonics may be derived by the smart electrical meter 100 or the non-invasive monitoring unit 400.

The communication module according to Fig. 4 is further configured to receive or acquire (S20) second output signals 20 from a smart water meter 200 coupled to a water supply of the residence, wherein the second output signals 20 indicate one or more usage parameters related to water appliances 210₁, 210₂, ..., 210_{M} connected to the water supply. Here, water appliances refer to any water-consuming appliance such as a kitchen sink, a shower, a lavatory or the like.

Usage parameters related to water appliances may refer to one or more of water consumption, a water flow rate, a flow velocity, a time, a time stamp, a usage time, a state (on, off, stand-by, maintenance) and the like. Such usage parameter may be determined by the water meter 200, for example based on ultrasound technology or remanent magnetic field technology. Here, the water meter 200 maybe a (smart) water meter that is connected to the water appliances 210₁, 210₂, ..., 210_{M} in the residence and to the waterworks, records the water consumption based on a variable sampling frequency (as small as minutes), and communicates corresponding information to the non-invasive monitoring unit 400. Furthermore, the water meter 200 may provide precise measurements of small flow rates, for example of 10 l/h, 1 l/h, 0.1 l/h or even smaller.

The communication module 420 might serve as an I/O, provide general connectivity to sensors, meters, and cloud services (which possibly includes Software as a Service (SaaS) or other services), provide information for maintenance (from extern). It might be wireless or wire-based.

The processing module 420 according to Fig. 4 is configured to process (S40) the first and second output signals based on one or more machine learning algorithms to classify individual electrical appliance usage and individual water appliance usage. Here, the first output signals 10 have a sampling frequency of at least 10 kHz. That is, based on the first output signals from the electrical meter 10 and the second output signals from the water meter 20, the usage of individual electrical appliances and the usage of individual water appliances is determined; for example whether a TV is used, whether an electric oven is used, whether a shower is used, whether the lavatory is used and the like. Further, based on the first output signals from the electrical meter 10 having a sampling frequency of at least 10kHz, operation modes of the individual electrical appliances may be determined; as will be further discussed below.

The sampling frequency may further vary or may be adapted or selected. In order to appropriately take the actual sampling frequency into account when classifying the individual electrical appliance usage, the non-invasive monitoring unit may be provided with current values of the sampling frequency, for example as a further part of the output signals 10, or may derive the actual sampling frequencies from data entries of the output signals 10, such as the time stamps.

Here, the electrical meter 100 may be a high-frequency non-instructive load monitoring smart meter (HNSM) for tracking electrical appliance usage within the monitored site (residence). Such an electrical meter 100 is capable of using maximum sampling frequencies of at least 10 kHz (as will be further described below). In particular, the smart electrical meter 100 may select a sampling frequency, for example 10 kHz, 50 kHz, 100 kHz depending on an input instruction provided by the non-invasive monitoring unit 400. As such, the sampling frequency may dynamically be selected or adapted, for example according to different conditions, as will be further described below. Here, the usage of such sampling frequencies allows for a suitable and improved classification of an individual appliance usage and therefore an improved classification of the user activity. More specifically, these higher sampling frequencies not only allow to distinguish/classify between individual electrical appliances, but also allow to distinguish/classify, for example, between different operating modes of an electrical appliance, e.g. different operating modes of an electrical oven (cooking, baking, grilling), different operating modes of a TV (frequent channel switching, internet usage, radio channel usage, TV station channel usage), and the like.

Note that the one or more machine learning algorithms themselves may be provided externally from the non-invasive monitoring unit 400. In particular, training, acquisition of training data (e.g. usage parameter as described above), and the like may be provided in an external data server (not shown) which provides a trained classification algorithm to the non-invasive monitoring unit 400. In other words, the non-invasive monitoring unit 400 may apply a trained classification algorithm in step S40 to classify the individual electrical appliance usage and/or individual water appliance usage.

Classification of individual electrical appliance usage based on one or more machine learning algorithms means that the acquired data of individual electrical appliance usage may be classified in such a way that non-invasive information is derived as to a particular usage of one or more electrical appliances by the user. In the simplest case, this might be realized by classifying the individual electrical appliance usage according to predefined categories such as "no electrical appliance usage", "low electrical appliance usage", "high electrical appliance usage", or categories such as "periodic/repeating electrical appliance usage", but it is not limited thereto. In a more advanced case this might lead to classification "electrical appliance usage typical for a TV, further indication towards use via laptop", which is only possible through the high sampling frequency provided by the high frequency smart electrical meter. If the used sampling frequency is high enough it is not only possible to deduce which devices are used but also in which operation mode they are used, such as which TV station is tuned into, etc. providing further information that is potentially crucial in order to determine activity (or non-activity) of a user. Consequently, categories such as "usage of device X", "usage of device X in combination with device Y", "usage of device X in mode Z" and the like could be determined. Further, the classification is not limited to the case of predefined categories. In particular, new categories might be added to the list of categories, for example if the usage parameters related to electrical appliances indicate parameters related to one or more of voltage, current, real and reactive power, harmonics, electromagnetic interference that appear to be related to a new electrical apparatus. Here, such usage parameter may be known to the machine learning algorithm / trained classification algorithm (for example, if trained in an external server), and thus detect a specific electrical apparatus that is newly purchased by the user and is used for the first time in the residence. Further, categories may be deleted if deemed necessary, for example if the categories are not used (any more) and/or different categories exist that provide better results. Further, if unknown usage parameters of an electrical appliance are detected, this may indicate a potential hazardous condition and the user or remote caregiver may be warned accordingly.

Similarly, classification of individual water appliance usage based on one or more machine learning algorithms means that the acquired data of individual water appliance usage may be classified in such a way that non-invasive information is derived as to a particular usage of one or more water appliances by the user. This might be realized by classifying the individual electrical appliance usage according to predefined categories such as "no water appliance usage", "low water appliance usage", "high water appliance usage", or categories such as "periodic/repeating water appliance usage", but it is not limited thereto. Further, also this classification is not limited to the case of predefined categories. In particular, new categories might be added to the list of categories, for example. In a more advanced case this might lead to classification "water appliance usage typical for a bathtub" or the like, possible through the higher sampling frequency provided by the smart water meter. In addition, classifications such as "water appliance usage typical for a bathtub and a sink" might be possible as well. Consequently, categories such as "usage of device X", "usage of device X in combination with device Y" and the like could be determined. Also, here categories may be deleted if deemed necessary, for example if the categories are not used and/or different categories exist that provide better results. Further, if usage parameters of a water appliance indicate water leaks, reverse water flows, or the like, this may also indicate a potential hazardous condition and the user or remote caregiver may be warned accordingly.

Furthermore, the classification may be combined in such a way that the usage parameters of both, electrical and water, appliance are combined. Examples for this might be a classification such as "water appliance usage typical for a bathtub" and "electrical appliance usage typical for an electrical stove". Such a classification may provide an improved classification of an activity or event (as will be further described below) . Such a classification may also be useful as an indication that something is not in order, for example that the bathtub might overflow the bathroom while the user is cooking in the kitchen or that on the stove food is burning while the user is taking a bath. These classifications are only possible with a high frequency electrical meter as only with such a high frequency the classification can be made accordingly. Without a high frequency electrical meter, the use of electricity and water at the same time, such as in the above example, could not be distinguished from a normal and unproblematic case such as "water appliance usage typical for a bathtub and electrical appliance usage typical for a radio", which would rather point to normal, harmless use of a listening to a radio while taking a bath.

The processing module 420 as depicted in Fig. 4 is further configured to, based on the one or more machine learning algorithms, classify (S50) an activity of the user and/or classify an event in the residence based on the classified electrical and water appliance usage.

Classification of an activity based on one or more machine learning algorithms means that the machine learning algorithm or a classification algorithm is able to deduce from the classification of the electrical appliance usage and from the classification of the water appliance usage that a certain activity using devices using electricity and/or water is performed. Activity of a user may generally refer to the usage and the change in the usage of a device. This can possibly include the usage of a combination of devices. As an example, such a classification might be, regarding electricity using devices, "Person is using the TV" or "Person is not using the computer anymore" or "Person switched on the lights in the bathroom", or, regarding water using devices, "Person fills the bathtub", or "Person empties out the water in the sink" or "Person uses the toilet", whereas activities might also include examples combining devices, such a "using the shower and using the radio" or "using the bathtub and the electrical stove".

Similarly, classification of an event based on one or more machine learning algorithms means that the machine learning algorithm is able to deduce from the classification of the electrical appliance usage and from the classification of the water appliance usage that a certain event involving devices using electricity and/or water is occurring. As an example, such a classification might be, regarding electricity using devices, "the light in the bedroom is still on", "the stove is still on and it is past midnight", or, regarding water using devices, "the water in the bathtub is still flowing so it might overflow" and the like.

As outlined above, the use of smart meters, in particular of a smart electrical meter with a high sampling frequency can be used to support old, handicapped or sick people, in particular regarding the use of places that are prone to accidents, such as the bathroom and the kitchen, which are particularly important.

Furthermore, the processing module 420 might be configured to, based on the one or more machine learning algorithms, predict (S60) an activity pattern of the user and/or an event pattern in the residence, to define (S70) rules based on the identified activity patterns and/or event patterns, to monitor (S80) the output signals and to apply (S80) the rules to the output signals to identify conditions that violate the rules.

Predicting an activity or event pattern means that from a plurality of classified activities or events, based on the one or more machine learning algorithms, a typical series of activities or events at a specific time, such as daily or weekly, and at a specific place, such as in a specific room in the residence, of a routine of the user is determined. This may include a typical order in a series of activities or events that represent a typical behavior of the user and/or a typical series of events occurring in the residence during the course of the day. This might include activities or events that occur at a specific time and place. An example of this might be "User gets up at 7 am, goes to the bathroom for 15 minutes and then has breakfast for half an hour."

For this pattern recognition, predefined patterns may be used which may include predefined patterns that have a certain degree of freedom suitable to be fit to the classified activities/events. An example for this might be "User gets up at XX am, goes to the bathroom for YY minutes and then has breakfast for ZZ minutes." or alternatively "User gets up at XX am, goes to the bathroom for YY minutes and is then expected to have breakfast for ZZ minutes." Here, the specific values for XX, YY, and ZZ are monitored over an extended period of time, for example several months. This allows for more variability and therefore predicted patterns that fit better to the user's behavior.

Further, the patterns may be updated regularly, that is weekly, daily, hourly or even more frequent, in order to provide optimal monitoring capabilities. Updating patterns might be due to newly acquired classification results, contexts or to alternative, external reasons such as a command by the user.

The step of defining rules, based on the identified activity patterns and/or event patterns, might include specific rules such as "the user gets up by 9am" or might include more flexible rules including time intervals, usage intervals, or the like, such as "the user gets up by between 7 and 8 am", "the user showers for 20 to 30 minutes". The rules may therefore include time conditions, that is at what time an activity or event typically occurs, and/or usage conditions, that is which and how one or more appliances are used. Then, based on the machine learning algorithm, it is possible to dynamically select among several rules that fit best to describe a normal or typical course of activities/events. That is, distinguishing between activities/events at different times may lead to different rules to be applied. For example, getting up at 7 am may typically lead to a different daily routine (i.e. a different rule) then getting up at 9 am.

Situations identified by the monitoring of output signals and applying the rules to these monitored signals may include hazardous situations, emergency situations and the like. That is, having identified the rules based on a series of previous output signals from the electrical meter 100, the water meter 200, and optionally a sensor 300, the non-invasive monitoring unit subsequently compares current output signals with time conditions, usage conditions and the like defined by the rules in order to determine whether the user(s) follow a typical routine or not. Not following a typical routine may indicate an abnormal or unusual behavior which may indicate an emergency situation which requires a check by a caregiver or a medical doctor.

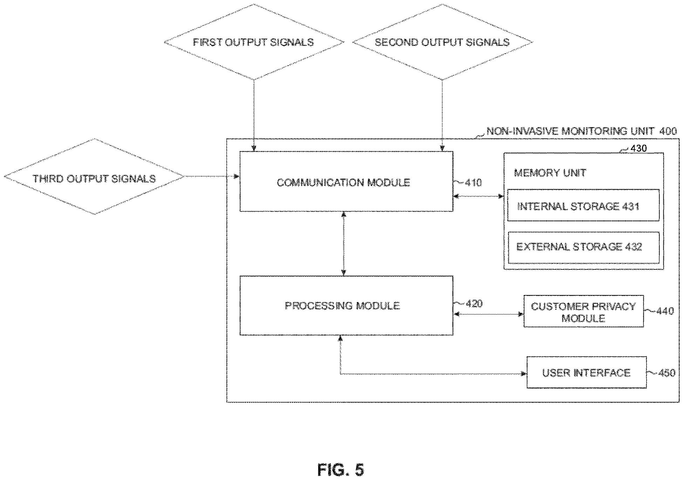

The processing module 420 as depicted in Fig. 5 might further be configured to adapt (S90) the used sampling frequency of the electric meter, preferably according to at least one of measured output signals, classified activities, and time of the day.

Adapting the used sampling frequency of the electrical meter 100 changes the sampling frequency of the first output signals that are used in the further steps as described herein. Adapting the used sampling frequency is useful because adapting high sampling frequencies of at least 10 kHz generate high numbers of data records which leads to disadvantageous data storage requirements.

This step of adapting the used sampling frequency of the electrical meter 100 can be influenced by various sources. Examples of such sources, namely the adapting or selection of the sampling frequency according to or based on the first and/or second output signals may be the first and/or second output signals themselves indicating that the usage of electricity and/or water is very low (indicating, for example, that the user is presently not in the residence) so that a classification or monitoring is not necessary. In such a case, the sampling frequency may be dynamically reduced in order to minimize the number of data records. On the other hand, if the first and/or second output signals indicate that the usage of electricity and/or water starts to increase (again), for example when a user has returned to the residence and switches on a light and/or washes hands, then the sampling frequency may be dynamically increased in order to continue the classification and monitoring steps.

Alternatively, or additionally, the adapting or selection of the sampling frequency may also be based on a classified activity or a classified event. For example, a specific activity (for example in an activity pattern) may indicate that the user will continue performing this activity for an extended period of time, such as typically watching TV between 7 PM and 9 PM. According to such a specific activity, no further activity is typically expected for the user, so that the sampling frequency may be dynamically reduced in order to minimize the number of data records.

Note however, that it is not limited to these, as other sources may be considered also. Other examples of such other sources are the specific time of a day (day/night, morning/midday/evening, etc.), established or predetermined routines of the user, alternative information acquired through other means, such as the presence of an additional person, that can have a considerable impact on the electrical and/or water supply and the capabilities of the method in general. During the night, for example, from 11 PM - 6 AM the following morning, the user typically sleeps so that it is not useful to keep the sampling frequency high. The sampling frequency may then be increased (again) around the typical wake-up time of the user, or based on a series of classified activities around a morning routine of the user.

The above adaptation or selection of the sampling frequency thus provides a feedback mechanism with regard to a dynamic variation of the sampling frequency that reduces the storage requirements and applies sampling frequencies of at least 10 kHz only in times of relevant user activity or to times when monitoring of the ALS is required.

This can be realized either directly in the electrical meter (for example, if the non-invasive monitoring unit is implemented in the electrical meter) or, if necessary, by a module of the non-invasive monitoring unit outside of the electrical meter.

Possible mechanisms on how to adapt the sampling frequency have already been established, for example selecting and applying a granularity level to the usage data and produce down-sampled usage data. This can in particular be realized without compromising the privacy of the user and further allowing the costumer to flexibly control the amount of information shared externally. Specific examples might include down-sampling by implementation of a binary-tree based scheme, e.g. a modified Merkle hash tree, or by implementation of a N-ary-tree (instead of binary) based scheme in order to achieve granularity of the data by means of subsequent hash value manipulation. This step can be made for various uses, e.g. data reduction for improved data transfer and improved data storage, feedback and privacy concerns.

In Fig. 5 another embodiment of the non-invasive monitoring unit 400 is depicted. Therein, the communication module 410 is further be configured to receive or acquire (S30) third output signals 30 of a sensor 300 of a residence. These third output signals 30 may, for example, refer to a gas appliance usage. These third output signals 30 may also refer to position information of the user, for example based on wearable devices which the user wears around the arm, the wrist, the neck or the like.

The processing module 420 might be further configure to use the third output signals 30 in at least one of the classifying (S40) individual appliance usage acquired from the sensor, the classifying (S50) the activity , the predicting (S60) the activity and/or event pattern, the defining (S70) rules based on the identified activity patterns and/or event patterns, and the monitoring (S80) the output signals and applying the rules to the output signals to identify situations that violate the rules.

Furthermore, the non-invasive monitoring unit 400 according to Fig. 5 comprises a memory unit 430 including an internal storage unit 431 and an external storage unit 432. The memory unit 430 may in particular store the data that has to be stored based on legal requirements, such as, for example, total power usage and water usage data for the European Energy Exchange (EEX) and their counterparts for water appliance. Further, the memory unit 430 may be divided into a short and long term memory storage. Here, the short term storage preferably stores raw data from the electrical meter and the water meter to a certain extent (for example depending on how much storage capacity is allocated for that use). The long term storage, on the other hand, preferably stores the time series data that result from the machine learning classification of activities, context etc. The long term storage may also store raw data that were deemed to be interesting for training the machine learning algorithm (on device) or which the customer wanted to share (on server).

The memory unit 430 stores raw data acquired from the various smart meter and other sensors and the like, further stores the results of the classification, context generation and prediction as well as other data that might be acquired during the process of monitoring.

Data stored in the external storage unit is data that the user has selected as releasable data, data that is released for third party access and the like.

In addition, the non-invasive monitoring unit 400 according to Fig. 5 comprises a customer privacy module 440.

The customer privacy module 440 protects data such as the private data, i.e. non-releasable data, from malicious activity. This can be realized by granularity modification, anonymization, encryption or the like. Here, the private data are to be distinguished from the power usage and water usage data which have to be shared (for example with the EEX which have to be shared every 15 minutes). In other words, the utility providers will have access to these types of utility data regardless of the user's privacy configuration.

Further, the non-invasive monitoring unit 400 according to Fig. 5 comprises a user interface module 450 configured to allow the user to selectively transmit data (step S100).

The transmitted data may be data generated by any of the steps S40, S50, S60, and S70 to a remote server, possibly a cloud server. The remote server (not shown) may implement the one or more machine learning algorithms, and receiving the data from a plurality of residence may improve the training of an actual classification algorithm that may subsequently be implemented or updated at local non-invasive monitoring units. Here, the transmitted data may be prepared by a processing module 420 of the non-invasive monitoring unit 400 in a way that is usable by the machine learning algorithm(s). In particular, the data may be transmitted via a dataset avoiding empty and inconsistent data entries which improves the machine learning process. The data transmitted might thus allow for further improvement of the machine learning algorithm(s). Data transmission may occur over a cloud infrastructure, over a private or public network and the like, and may be based on, for example, private and public keys, in order to provide data security. In addition, the user of the residence may select, for example via a graphical user interface, which of the data are to be transferred to the remote server in order to provide a data privacy mechanism. This might in particular be realized in cooperation with the privacy customer module 440.

Preferably, the user may select the granularity, frequency and/or modulate access permissions for the transmitted data. For example, if the user decides to partake in, for example, a research study or has agreed to train the machine learning algorithms (which will be conducted in external servers), full access can be given, either to the server or a third party. This includes possibly full access to the raw, classified and activity data. This data will be transferred onto an external server for further processing. To ensure data privacy, activity timelines and context classification will be handled and classified (using machine learning) on devices only, unless agreed to the conditions above.

The user interface module, also called a human-machine interface, might be a display, might be touch-based or haptic. It might allow for graphical user interaction and might allow direct access to the data, classifications, context, predictions and the like acquired by the meters and generated by the machine learning algorithms.

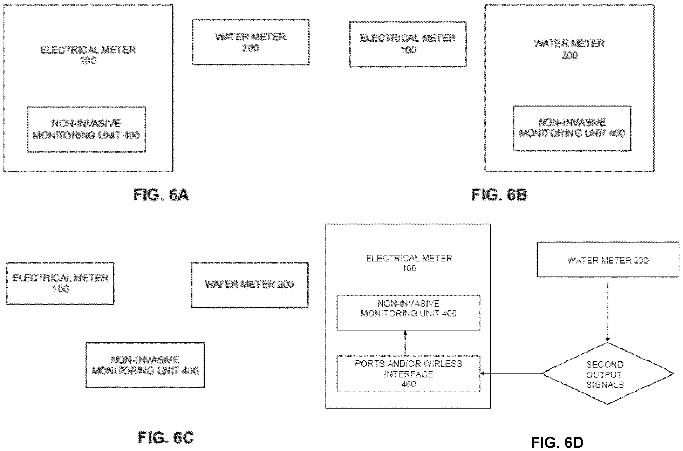

Next, Fig. 6 shows four possible embodiments of the non-invasive monitoring unit 400.

According to Fig. 6A the non-invasive monitoring unit 400 might be embedded into the electrical meter 100. According to Fig. 6B the non-invasive monitoring unit 400 might be embedded into the water meter 200. According to Fig. 6C the non-invasive monitoring unit 400 might be a standalone unit, separate from the electrical meter 100 and the water meter 200. It is not limited thereto; in particular it might be embedded into another sensor unit, if present. Further, according to Fig. 6D the non-invasive monitoring unit 400 might be embedded into the electrical meter 100 and the smart electrical meter 100 might have ports and/or a wireless interface 460 to receive the second output signals 20 of the water meter 200. This provides a mechanism to import and/or export data into and/or out of the meters, and is equally used in the context of the embodiments of Figs. 6B and 6C.

## Claims

1. A computer-implemented method for non-invasive monitoring, the method comprising:
- receiving (S10) first output signals (10) from an electrical meter (100) coupled to an electrical supply of a residence, the first output signals (10) indicating one or more usage parameters related to electrical appliances (110₁, 110₂, ... 110_{N}) connected to the electrical supply;
- receiving (S20) second output signals (20) from a water meter (200) coupled to a water supply of the residence, the second output signals (20) indicating one or more usage parameters related to water appliances (120₁, 120₂, ... 120_{M}) connected to the water supply;
- processing (S40) the first (10) and second output signals (20) based on one or more machine learning algorithms to classify individual electrical appliance usage and individual water appliance usage; the first signals having a sampling frequency of at least 10 kHz; and
- based on the one or more machine learning algorithms, classifying (S50) an activity of a user and/or classifying an event in the residence based on the classified individual electrical and water appliance usage.

2. The computer-implemented method according to claim 1, further comprising the step of:
- based on the one or more machine learning algorithms, predicting (S60) an activity pattern of the user and/or an event pattern in the residence.

3. The computer-implemented method according to claim 2, further comprising:
- defining (S70) rules based on the identified activity patterns and/or event patterns; and
- monitoring (S80) the output signals and applying the rules to the output signals to identify situations that violate the rules.

4. The computer-implemented method according to any one of claims 1 to 3, further comprising:
- adapting (S90) the used sampling frequency of the electrical meter (100).

5. The computer-implemented method according to any one of claims 1 to 4, wherein the used sampling frequency is adapted based on at least one of
- the first output signals (10) and/or the second output signals (20),
- the classified activity and/or the classified event, and
- a time of the day.

6. A non-invasive monitoring unit (400), comprising:
a communication module (410), configured to
- receive (S10) first output signals (10) of a electrical meter (100) coupled to an electrical supply of a residence, the first output signals (10) indicating one or more usage parameters related to electrical appliances connected to the electrical supply; and
- receive (S20) second output signals (20) of a water meter (200) coupled to a water supply of the residence, the second output signals (20) indicating one or more usage parameters related to water appliances connected to the water supply;
a processing module (420), configured to:
- process (S40) the first (10) and second output signals (20) based on one or more machine learning algorithms to classify individual electrical appliance usage and individual water appliance usage, the first signals having a sampling frequency of at least 10kHz; and
- based on the one or more machine learning algorithms, classify (S50) an activity of the user and/or classify an event in the residence based on the classified electrical and water appliance usage.

7. The non-invasive monitoring unit (400) according to claim 6, wherein the processing module is further configured to, based on one or more machine learning algorithms, predict (S60) an activity pattern of the user and/or an event pattern in the residence.

8. The non-invasive monitoring unit (400) according to claim 7, wherein the processing module is further configured to define (S80) rules based on the identified activity patterns and/or event patterns, and is further configured to monitor the output signals and apply the rules to the output signals to identify conditions that violate the rules.

9. The non-invasive monitoring unit (400) according to any one of claims 6 to 8, wherein the processing module is further configured to adapt (S90) the used sampling frequency of the electric meter.

10. The non-invasive monitoring unit (400) according to claim 9, wherein the used sampling frequency is adapted based on at least one of
- the first output signals (10) and/or the second output signals (20),
- the classified activity and/or the classified event, and
- a time of the day.

11. The non-invasive monitoring unit (400) according to any one of claims 6 to 10, further comprising a user interface module (450) configured to allow the user to selectively transmit data, preferably by selecting a granularity, a frequency and/or modulate access permissions for the transmitted data.

12. The non-invasive monitoring unit (400) according to any of claims 6 to 11, further comprising a memory unit (430) including an internal storage unit (431) and an external storage unit (432).

13. The non-invasive monitoring unit (400) according to any of claims 6 to 12, further comprising a customer privacy module (440).

14. The non-invasive monitoring unit (400) according to any of claims 6 to 13, wherein the non-invasive monitoring unit (400) is embedded into the electrical meter (100), the water meter (200), another sensor unit (300), or is a standalone unit.

15. The non-invasive monitoring unit (400) according to any of claims 6 to 14, wherein the non-invasive monitoring unit (400) is embedded into the electrical meter (100) and wherein the electrical meter (100) has ports and/or a wireless interface to receive the second output signals (20) from the water meter (200).

## Amended claims

### Amended claims in accordance with Rule 137(2) EPC.

1. A computer-implemented method for non-invasive monitoring, the method comprising:
- receiving (S10) first output signals (10) from an electrical meter (100) coupled to an electrical supply of a residence, the first output signals (10) indicating one or more usage parameters related to electrical appliances (110₁, 110₂, ... 110_{N}) connected to the electrical supply;
- receiving (S20) second output signals (20) from a water meter (200) coupled to a water supply of the residence, the second output signals (20) indicating one or more usage parameters related to water appliances (120₁, 120₂, ... 120_{M}) connected to the water supply;
- processing (S40) the first (10) and second output signals (20) based on one or more machine learning algorithms to classify individual electrical appliance usage and individual water appliance usage by applying a trained classification algorithm; the first signals having a sampling frequency of at least 10 kHz; and
- based on the one or more machine learning algorithms, classifying (S50) an activity of a user and/or classifying an event in the residence based on the classified individual electrical and water appliance usage by deducing from the classification of the electrical appliance usage and from the classification of the water appliance usage that a certain activity and/or event involving devices using electricity and/or water is occurring,
wherein the used sampling frequency is adapted based on at least one of
- the classified activity and/or the classified event, and
- a time of the day.

2. The computer-implemented method according to claim 1, further comprising the step of:
- based on the one or more machine learning algorithms, predicting (S60) an activity pattern of the user and/or an event pattern in the residence by using predefined patterns including predefined patterns that have a certain degree of freedom suitable to be fit to the classified activities/events.

3. The computer-implemented method according to claim 2, further comprising:
- defining (S70) rules based on the identified activity patterns and/or event patterns; and
- monitoring (S80) the output signals and applying the rules to the output signals to identify situations that violate the rules.

4. The computer-implemented method according to any one of claims 1 to 3, further comprising:
- adapting (S90) the used sampling frequency of the electrical meter (100).

5. A non-invasive monitoring unit (400), comprising:
a communication module (410), configured to
- receive (S10) first output signals (10) of a electrical meter (100) coupled to an electrical supply of a residence, the first output signals (10) indicating one or more usage parameters related to electrical appliances connected to the electrical supply; and
- receive (S20) second output signals (20) of a water meter (200) coupled to a water supply of the residence, the second output signals (20) indicating one or more usage parameters related to water appliances connected to the water supply;
a processing module (420), configured to:
- process (S40) the first (10) and second output signals (20) based on one or more machine learning algorithms to classify individual electrical appliance usage and individual water appliance usage by applying a trained classification algorithm, the first signals having a sampling frequency of at least 10kHz; and
- based on the one or more machine learning algorithms, classify (S50) an activity of the user and/or classify an event in the residence based on the classified electrical and water appliance usage by deducing from the classification of the electrical appliance usage and from the classification of the water appliance usage that a certain even involve devices using electricity and/or water is occurring,
wherein the used sampling frequency is adapted based on at least one of
- the classified activity and/or the classified event, and
- a time of the day.

6. The non-invasive monitoring unit (400) according to claim 5, wherein the processing module is further configured to, based on one or more machine learning algorithms, predict (S60) an activity pattern of the user and/or an event pattern in the residence by using predefined patterns including predefined patters that have a certain degree of freedom suitable to be fit to the classified activities/events;

7. The non-invasive monitoring unit (400) according to claim 6, wherein the processing module is further configured to define (S80) rules based on the identified activity patterns and/or event patterns, and is further configured to monitor the output signals and apply the rules to the output signals to identify conditions that violate the rules.

8. The non-invasive monitoring unit (400) according to any one of claims 5 to 7, wherein the processing module is further configured to adapt (S90) the used sampling frequency of the electric meter.

9. The non-invasive monitoring unit (400) according to any one of claims 5 to 8, further comprising a user interface module (450) configured to allow the user to selectively transmit data.

10. The non-invasive monitoring unit (400) according to claim 9, wherein the selective transmission of data is achieved by selecting a granularity, a frequency and/or modulate access permissions for the transmitted data.

11. The non-invasive monitoring unit (400) according to any of claims 5 to 10, further comprising a memory unit (430) including an internal storage unit (431) and an external storage unit (432).

12. The non-invasive monitoring unit (400) according to any of claims 5 to 11, further comprising a customer privacy module (440) configured to protect data that is non-releasable data.

13. The non-invasive monitoring unit (400) according to any of claims 5 to 12, wherein the non-invasive monitoring unit (400) is embedded into the electrical meter (100), the water meter (200), another sensor unit (300), or is a standalone unit.

14. The non-invasive monitoring unit (400) according to any of claims 5 to 13, wherein the non-invasive monitoring unit (400) is embedded into the electrical meter (100) and wherein the electrical meter (100) has ports and/or a wireless interface to receive the second output signals (20) from the water meter (200).
